(19) Europäisches Patentamt
European Patent Office
Office européen des brevets

(11) **EP 3 046 327 A1**

(12) **EUROPEAN PATENT APPLICATION**
published in accordance with Art. 153(4) EPC

(43) Date of publication:
**20.07.2016 Bulletin 2016/29**

(51) Int Cl.:
*H04N 19/107* (2014.01)    *H04N 19/162* (2014.01)
*H04N 19/172* (2014.01)

(21) Application number: **14842832.9**

(22) Date of filing: **05.06.2014**

(86) International application number:
**PCT/JP2014/065000**

(87) International publication number:
**WO 2015/033635 (12.03.2015 Gazette 2015/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.09.2013 JP 2013186584**

(71) Applicant: **OLYMPUS CORPORATION**
**Shibuya-ku**
**Tokyo 151-0072 (JP)**

(72) Inventor: **NISHIMURA, Norio**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **IMAGE DISPLAY APPARATUS, ENCODING METHOD, AND ENCODING PROGRAM**

(57) An image display device 4 performs a playback operation to sequentially display multiple images on a display unit 44. The image display device 4 includes an input unit 43 that receives a designation of any playback position among multiple playback positions for starting the playback operation; a relating unit 451 that relates multiple playback positions to the number of images that are included in the multiple images and that correspond to the number of multiple playback positions; and an encoding unit 452 that compresses and encodes multiple images by using, as key frames, the images that are included in the multiple images and that are related to multiple playback positions by the relating unit 451 and by using a correlation between at least two of the images.

FIG.2

**Description**

Field

The present invention relates to an image display device that performs a playback operation to sequentially display multiple images on a display unit, an encoding method that is implemented by the image display device, and an encoding program that is executed by the image display device.

Background

Heretofore, capsule endoscope systems have been proposed in which an in-vivo image inside a subject is acquired by using a capsule endoscope that captures the inside of the subject, and the in-vivo image is observed by a doctor, or the like (for example, see Patent Literature 1).

After the capsule endoscope is swallowed through the mouth of the subject for observation (examination), it is moved in accordance with a peristaltic action inside a body cavity, e.g., inside an organ, such as a stomach or small intestine, until it is naturally excreted, and it captures the inside of the subject in accordance with the movement. Furthermore, while the capsule endoscope is moved inside the body cavity, it externally transmits the image data that is captured inside the body in sequence via a wireless communication.

Moreover, the capsule endoscope system includes a receiving device and an image display device in addition to the above-described capsule endoscope.

The receiving device sequentially receives the image data that is transmitted from the capsule endoscope and sequentially records it in a portable recording medium that is inserted into the receiving device.

When the above-described portable recording medium is inserted into the image display device, the image display device fetches the image data that is recorded in the recording medium. Then, the image display device displays (frame playback) the in-vivo image that corresponds to the fetched image data by switching it frame-by-frame. Furthermore, together with the display of the above-described in-vivo image, the image display device displays a time bar that indicates the total time after capturing of an in-vivo image is started until it is terminated and a slider for designating the position of the in-vivo image that is displayed on the time bar as described above. Here, when the slider is moved in response to a mouse operation, or the like, by a doctor, or the like, the image display device displays the in-vivo image that corresponds to the position of the slider on the time bar, thereby enabling what is called a random playback.

Furthermore, an enormous number (sixty thousands to one hundred thousand) of in-vivo images are captured by a capsule endoscope during a single examination.

Therefore, in consideration of the data capacity of an image display device, it is preferable that, after an in-vivo image is compressed by using a still-image compression technology, such as JPEG, or a moving-image compression technology, such as inter-frame predictive encoding, it is stored in a storage unit of the image display device.

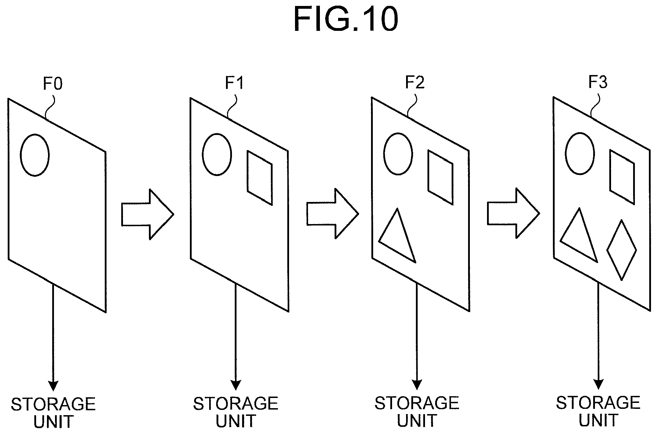

FIG. 10 is a diagram that illustrates a conventional still-image compression technology.

Furthermore, in FIG. 10, an image is indicated by "F". Moreover, the number that follows "F" indicates a frame number in chronological order in a case where all the images are virtually arranged in chronological order. The same holds for the drawings that are described below.

According to the conventional still-image compression technology, as illustrated in FIG. 10, multiple images (only four images F0 to F3 are illustrated in FIG. 10), which change in chronological order, are individually compressed and encoded and are stored in a storage unit. Therefore, the data compression rate cannot be increased.

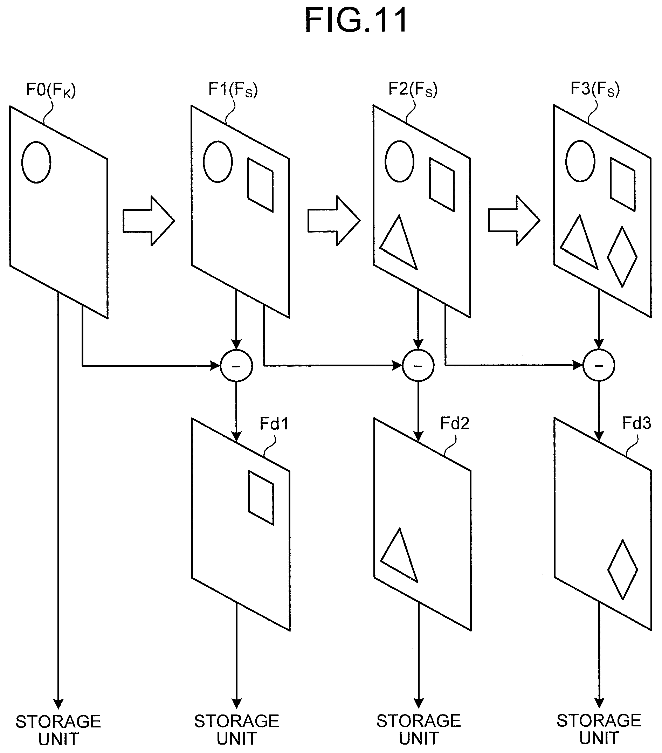

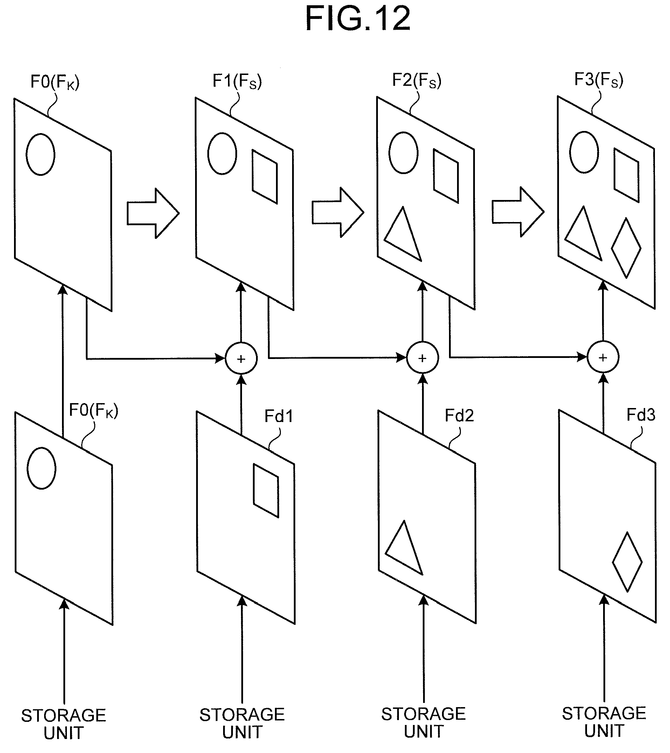

FIG. 11 and FIG. 12 are diagrams that illustrate a conventional moving-image compression technology.

Specifically, FIG. 11 and FIG. 12 are diagrams that illustrate a simple inter-frame predictive encoding method that is an example of the image compression encoding according to a moving-image compression technology. Furthermore, FIG. 11 is a diagram that illustrates image compression encoding, and FIG. 12 is a diagram that illustrates an image decoding operation.

Furthermore, according to the conventional moving-image compression technology, as illustrated in FIG. 11 or FIG. 12, among multiple images (only the four images F0 to F3 are illustrated in FIG. 11 or FIG. 12) that change in chronological order, the image that is located at an arbitrary position is a key frame $F_K$ (the image F0 in FIG. 11 or FIG. 12), and the other images are non-key frames $F_S$ (the images F1 to F3 in FIG. 11 or FIG. 12).

Then, according to the moving-image compression technology, compression encoding is performed as described below (FIG. 11).

The key frame $F_K$ is directly compressed and encoded and is stored in the storage unit. Furthermore, with regard to the non-key frames $F_S$, subtraction is performed with the key frame $F_K$ that is previous in chronological order or the non-key frame $F_S$, and the subtracted image (in FIG. 11, a subtracted image Fd1 between the image F0 and the image F1, a subtracted image Fd2 between the image F1 and the image F2, and a subtracted image Fd3 between the image F2 and the image F3) is compressed and encoded and is stored in the storage unit.

Furthermore, the following decoding operation is performed (FIG. 12) to decode the image that is compressed and encoded as described above.

With regard to the key frame $F_K$, the compressed and encoded key frame $F_K$ is read from the storage unit and is subjected to a decoding operation. Moreover, the non-key frame $F_S$ is generated such that the compressed and encoded subtracted image is read from the storage unit and is subjected to a decoding operation and then the subtracted image is combined with the decoded key frame $F_K$ that is previous to the non-key frame $F_S$ in chronological order or the non-key frame $F_S$.

Citation List

Patent Literature

Patent Literature 1: Japanese Patent No. 5197892 Summary

Technical Problem

The above-described compression encoding is performed according to the moving-image compression technology; therefore, the data compression rate can be increased compared to a still-image compression technology in which multiple images are individually compressed and encoded.

However, according to the moving-image compression technology, the above-described decoding operation is performed and therefore there is the following problem when a random playback is performed as described in Patent Literature 1.

If a playback designation for playback from the position (playback position) of the key frame $F_K$ is received, a decoding operation may be performed on the key frame $F_K$ that is compressed and encoded as described above to display the image at the playback position; therefore, it does not take time from when the playback designation is received until when the image (key frame) at the playback position is displayed.

Conversely, in a case where a playback designation for playback from the position (playback position) of the non-key frame $F_S$ is received, in order to display the image (non-key frame) at the playback position, the decoded key frame $F_K$ that is previous to the image in chronological order or the non-key frame $F_S$ is needed, as described above. For example, if a playback designation for playback from the position (playback position) of the non-key frame F3 illustrated in FIG. 12 is received, the key frame F0 is first decoded to display the non-key frame F3 at the playback position. Next, the non-key frame F1 is generated by using the key frame F0. Next, the non-key frame F2 is generated by using the non-key frame F1. Then, when the non-key frame F2 is generated, the non-key frame F3 can be generated for the first time. Therefore, there is a problem in that it takes time from when the playback designation is received until when the image (non-key frame) at the playback position is displayed.

The present invention has been made in consideration of the foregoing, and it provides an image display device, an encoding method, and an encoding program that make it possible to promptly display images at various playback positions by using a moving-image compression technology that has a higher data compression rate compared to a still-image compression technology.

Solution to Problem

To solve the problem described above and to achieve the object, an image display device according to the invention performs a playback operation to sequentially display multiple images on a display unit. The image display device includes: a designation receiving unit that receives a designation of any playback position among multiple playback positions for starting the playback operation; a relating unit that relates the multiple playback positions to the number of the images that are included in the multiple images and that correspond to the number of the multiple playback positions; and an encoding unit that compresses and encodes the multiple images by using, as key frames, images that are included in the multiple images and that are related to the multiple playback positions by the relating unit and by using a correlation between at least two of the images.

In the above-described image display device, the relating unit uses the number of the multiple playback positions and entire-volume information that indicates a volume of the multiple images in entirety to relate the multiple playback positions to the number of the images that are included in the multiple images and that corresponds to the number of the multiple playback positions.

In the above-described image display device, the entire-volume information is the number of frames of the multiple images in entirety, and the relating unit calculates an index value Kp by using $Kp=(p \cdot (M-1))/(N-1)$, where the number of frames is M, the number of the multiple playback positions is N, and an index value that indicates an image that is related to the p (p=0, 1, ..., N-1)th playback position is Kp, and relates an image with a frame number that is closest to the index value Kp to the pth playback position.

In the above-described image display device, the multiple images are images that are acquired in chronological order and that are associated with an elapsed time after corresponding acquisition is started, the entire-volume information is a total time after the acquisition of the multiple images is started until terminated, and the relating unit calculates an index value Kp' by using Kp'=(p·M')/(N-1), where the total time is M', the number of the multiple playback positions is N, and the index value that indicates an image that is related to the p (p=0, 1, ..., N-1)th playback position is Kp', and relates an image that is associated with the elapsed time that is closest to the index value Kp' to the pth playback position.

In the above-described image display device, the image display device includes: a decoding unit that decodes the multiple images that are compressed and encoded by the encoding unit; and a display controller that performs the playback operation on the multiple images that are decoded by the decoding unit and that displays, on the display unit, a bar that indicates a volume of the multiple images in entirety. The number of the playback positions provided corresponds to the number of pixels in a length direction of the bar that is displayed on the display unit.

In the above-described image display device, the display controller displays, on the display unit, a playback screen that includes the image and the bar and is capable of changing a resolution of the playback screen to multiple resolutions, the image display device includes a memory unit that stores the number of the playback positions that correspond to each of the multiple resolutions, and the relating unit reads, from the memory unit, the number of the playback positions that correspond to the resolution of the playback screen that is displayed on the display unit and uses the number of the playback positions to relate the multiple playback positions to the number of the images that are included in the multiple images and that correspond to the number of the multiple playback positions.

An encoding method according to the invention an encoding method that performed by an image display device that performs a playback operation to sequentially display multiple images on a display unit. The encoding method includes: a relating step of relating multiple playback positions for starting the playback operation to the number of the images that are included in the multiple images and that correspond to the number of the multiple playback positions; and an encoding step of compressing and encoding the multiple images by using, as key frames, images that are included in the multiple images and that are related to the multiple playback positions at the relating step and by using a correlation between at least two of the images.

An encoding program according to the invention causes the image display device to execute the above-described encoding method.

Advantageous Effects of Invention

The image display device according to the present invention includes the relating unit and the encoding unit as described above. Therefore, when compression encoding is performed by the encoding unit, all the key frames can be the images that are related to multiple playback positions. Specifically, even if various playback positions are designated, as the image at the playback position is a key frame, the time it takes to display the image can be relatively short.

Therefore, with the image display device according to the present invention, an advantage is produced such that images at various playback positions can be promptly displayed by using a moving-image compression technology that has a higher data compression rate compared to a still-image compression technology.

As the encoding method according to the present invention is the method that is implemented by the above-described image display device, the same advantage as that of the above-described image display device can be produced.

As the encoding program according to the present invention is the program that is executed by the above-described image display device, the same advantage as that of the above-described image display device is produced.

Brief Description of Drawings

FIG. 1 is a schematic diagram that illustrates an image display system according to a first embodiment of the present invention.

FIG. 2 is a block diagram that illustrates the image display device illustrated in FIG. 1.

FIG. 3 is a diagram that illustrates an example of a playback screen that is displayed on a display unit that is illustrated in FIG. 2.

FIG. 4 is a flowchart that illustrates an encoding method according to the first embodiment of the present invention.

FIG. 5 is a diagram that illustrates Step S1B that is illustrated in FIG. 4.

FIG. 6 is a flowchart that illustrates an image display method according to the first embodiment of the present invention.

FIG. 7 is a diagram that illustrates Step S1B according to a second embodiment of the present invention.

FIG. 8 is a diagram that illustrates an example of the playback screen according to a third embodiment of the present invention.

FIG. 9A is a diagram that illustrates Step S1B according to the third embodiment of the present invention.

FIG. 9B is a diagram that illustrates Step S1B according to the third embodiment of the present invention.

FIG. 9C is a diagram that illustrates Step S1B according to the third embodiment of the present invention.

FIG. 10 is a diagram that illustrates a conventional still-image compression technology.

FIG. 11 is a diagram that illustrates a conventional moving-image compression technology.

FIG. 12 is a diagram that illustrates a conventional moving-image compression technology.

Description of Embodiments

A detailed explanation is given below, with reference to the drawings, of a preferred embodiment of an image display device, an encoding method, and an encoding program according to the present invention. Furthermore, the present invention is not limited to the embodiment.

(First embodiment)

[Schematic configuration of an image display system]

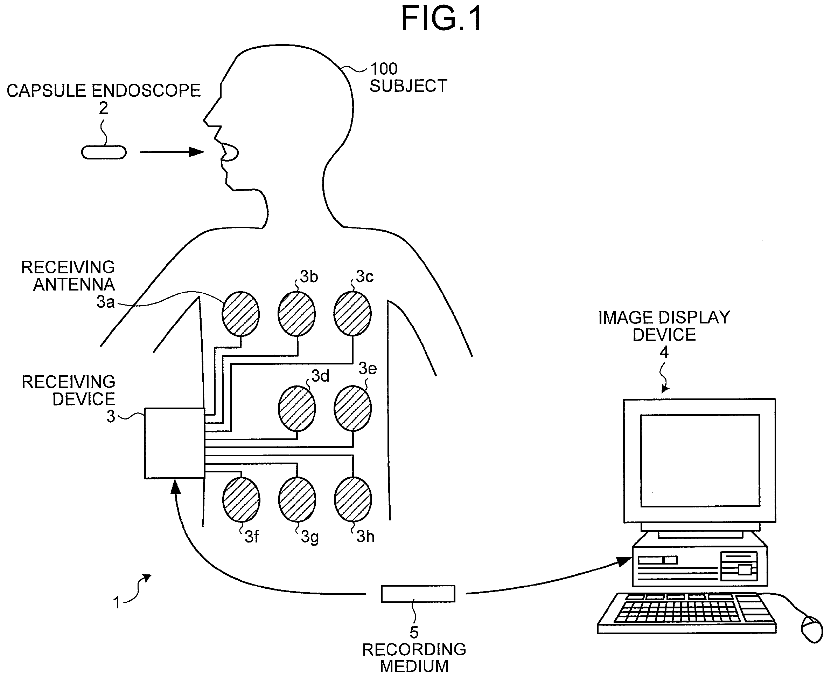

FIG. 1 is a schematic diagram that illustrates an image display system 1 according to a first embodiment of the present invention.

The image display system 1 is a system that uses a swallowable-type capsule endoscope 2 to acquire an in-vivo image inside a subject 100 and causes a doctor, or the like, to observe the in-vivo image.

As illustrated in FIG. 1, the image display system 1 includes a receiving device 3, an image display device 4, a portable recording medium 5, or the like, in addition to the capsule endoscope 2.

The recording medium 5 is a portable recording medium that transfers data between the receiving device 3 and the image display device 4, and it is configured to be attached to or detached from the receiving device 3 and the image display device 4.

The capsule endoscope 2 is a capsule endoscopic device that is formed in a size such that it can be inserted into inside of an organ of the subject 100, and it is orally ingested, or the like, so that it is inserted into inside of an organ of the subject 100 and is moved inside the organ due to a peristaltic action, or the like, while it sequentially captures in-vivo images. Furthermore, the capsule endoscope 2 associates the image data that is generated due to capturing with relevant information, such as the time (time stamp) after capturing starts or the frame number, and sequentially transmits the image data (including the relevant information).

Here, according to the first embodiment, the frame rate during capturing by the capsule endoscope 2 is fixed to a predetermined value.

The receiving device 3 includes multiple receiving antennas 3a to 3h, and it receives image data from the capsule endoscope 2 inside the subject 100 via at least one of the receiving antennas 3a to 3h. Then, the receiving device 3 stores the received image data in the recording medium 5 that is inserted into the receiving device 3. Furthermore, the receiving device 3 stores the entire-volume information that indicates the volume of the entire received image data in the recording medium 5 that is inserted into the receiving device 3.

Here, according to the first embodiment, the receiving device 3 stores the total number of frames of the received in-vivo image as the entire-volume information in the recording medium 5.

Furthermore, the receiving antennas 3a to 3h may be located on the body surface of the subject 100 as illustrated in FIG. 1 or may be located on a jacket that is worn on the subject 100. Moreover, the number of receiving antennas included in the receiving device 3 may be one or more, and it is not particularly limited to eight.

[Configuration of the image display device]

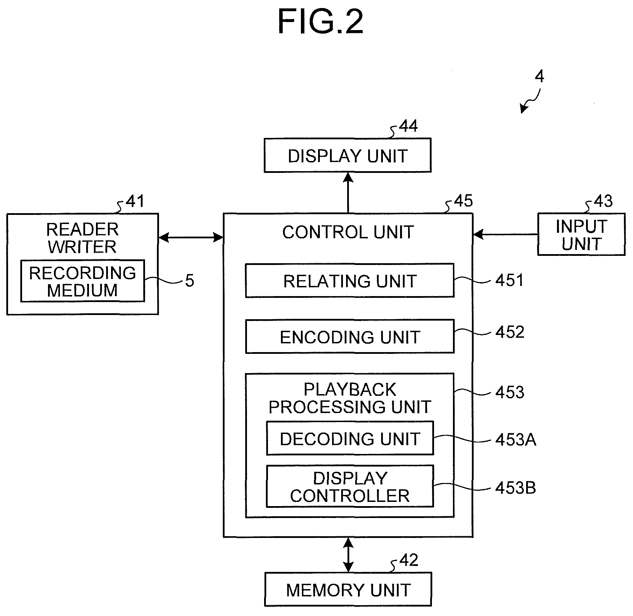

FIG. 2 is a block diagram that illustrates the image display device 4.

The image display device 4 is configured as a workstation that acquires image data on the inside of the subject 100 and displays the image that corresponds to the acquired image data.

As illustrated in FIG. 2, the image display device 4 includes a reader writer 41, a memory unit 42, an input unit 43, a display unit 44, a control unit 45, or the like.

When the recording medium 5 is inserted into the reader writer 41, the reader writer 41 fetches image data (an in-vivo image group that includes a plurality of in-vivo images that are captured (acquired) by the capsule endoscope 2 in chronological order) and the entire-volume information that are stored in the recording medium 5 under the control of the control unit 45. Moreover, the reader writer 41 transfers the fetched in-vivo image group or entire-volume information to the control unit 45. Then, the in-vivo image group, which has been transferred to the control unit 45, is compressed and encoded using the entire-volume information by the control unit 45 and is then stored in the memory unit 42.

The memory unit 42 stores the in-vivo image group that is compressed and encoded by the control unit 45.

Furthermore, the memory unit 42 stores various programs (including an encoding program) that is executed by the control unit 45 or information, or the like, that is needed for an operation of the control unit 45.

The input unit 43 is configured by using a keyboard, mouse, or the like, and it receives a user's operation by a doctor, or the like.

Furthermore, the input unit 43 has functionality as a designation receiving unit according to the present invention.

The display unit 44 is configured by using a liquid crystal display, or the like, and it displays a playback screen, or the like, that includes an in-vivo image under the control of the control unit 45.

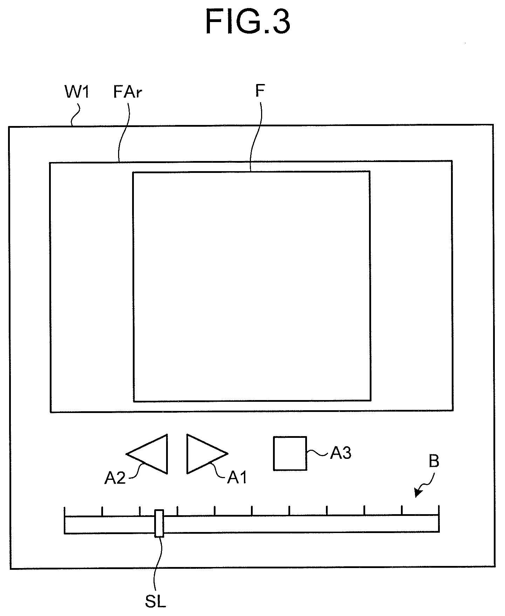

FIG. 3 is a diagram that illustrates an example of a playback screen W1 that is displayed on the display unit 44.

As illustrated in FIG. 3, the playback screen W1 is the screen where an image display area FAr, a forward-direction playback icon A1, a reverse-direction playback icon A2, a temporary stop icon A3, a time bar B, a slider SL, or the like, are arranged.

The image display area FAr is the area that displays the image F.

The forward-direction playback icon A1 is the icon that receives a playback designation (a forward-direction playback designation) for sequentially displaying (a forward-direction playback operation) in-vivo images on a frame-to-frame basis in a forward direction according to the chronological order.

The reverse-direction playback icon A2 is the icon that receives a playback designation (a reverse-direction playback designation) for sequentially displaying (a reverse-direction playback operation) in-vivo images on a frame-to-frame basis in the direction opposite to the forward direction.

The temporary stop icon A3 is the icon that receives a stop designation for displaying a still image by temporarily stopping a playback operation (a forward-direction playback operation and a reverse-direction playback operation).

The time bar B is a time scale that corresponds to the time from when the capsule endoscope 2 starts capturing until when capturing is stopped.

The slider SL designates the position (playback position) on the time bar B that temporally corresponds to the time stamp in the in-vivo image F that is displayed (played back) in the image display area FAr. Furthermore, the slider SL has a function to receive a designation for changing the playback position in accordance with a user's operation (for example, a mouse operation) on the input unit 43.

Here, the number of provided playback positions corresponds to the number of pixels in the length direction (in the horizontal direction in FIG. 3) of the time bar B (the number of pixels from the extreme left of the time bar B to the extreme right) in the playback screen W1. Specifically, the number of playback positions is a finite number. Information on the number of playback positions is previously stored in the memory unit 42.

Furthermore, the above-described slider SL is movable on the time bar B on a pixel by pixel basis in accordance with a user's operation on the input unit 43. Specifically, the slider SL receives a designation for changing the playback position on a pixel by pixel basis along the length direction of the time bar B.

The control unit 45 is configured by using a CPU (Central Processing Unit), or the like, and it reads a program (including an encoding program) stored in the memory unit 42 and controls the overall operation of the image display device 4 in accordance with the program.

As illustrated in FIG. 2, the control unit 45 includes a relating unit 451, an encoding unit 452, a playback processing unit 453, or the like.

On the basis of the number of multiple playback positions and the entire-volume information, the relating unit 451 relates the multiple playback positions to the number (the same number as the number of the playback positions) of in-vivo images that are included in the in-vivo image group and that correspond to the number of the multiple playback positions.

The encoding unit 452 compresses and encodes multiple in-vivo images by using, as key frames, the in-vivo images that are included in the in-vivo image group and that are related to the multiple playback positions by the relating unit 451 and by using the correlation between at least two in-vivo images, and it causes the memory unit 42 to store them.

The playback processing unit 453 displays the playback screen W1 in the display unit 44 and performs a playback operation (a forward-direction playback operation and a reverse-direction playback operation) to sequentially display the in-vivo image group stored in the memory unit 42 on a frame-to-frame basis in accordance with a user's operation on the input unit 43.

As illustrated in FIG. 2, the playback processing unit 453 includes a decoding unit 453A, a display controller 453B, or the like.

The decoding unit 453A reads an in-vivo image that is stored in the memory unit 42 and performs a decoding operation.

The display controller 453B displays the playback screen W1 in the display unit 44 and displays, on the image display area FAr in the playback screen W1, the in-vivo image on which a decoding operation has been performed by the decoding unit 453A.

[Operation of the image display device]

Next, an operation of the above-described image display device 4 is explained.

An explanation is sequentially given below of an encoding method and an image display method that are implemented by the image display device 4.

[Encoding method]

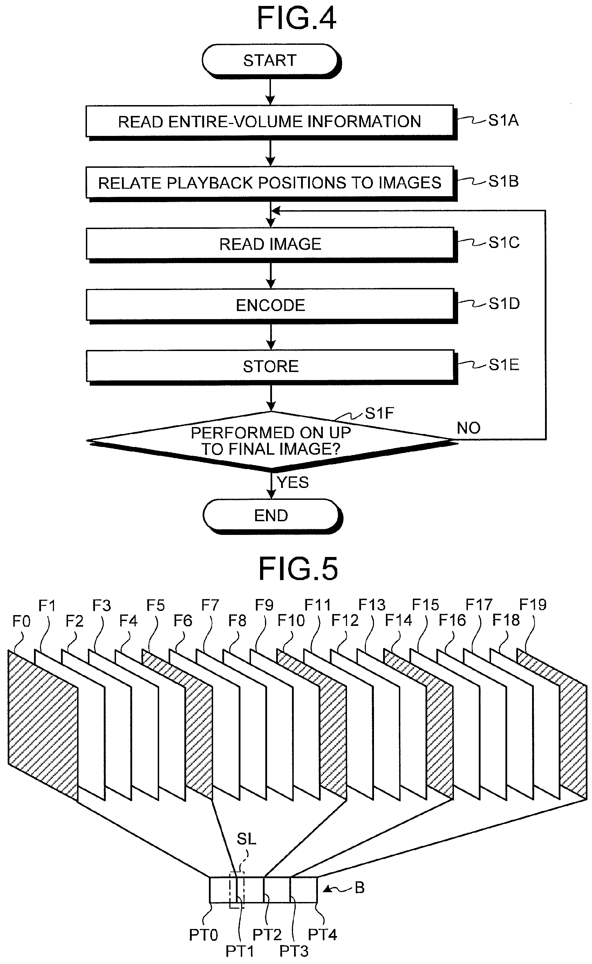

FIG. 4 is a flowchart that illustrates the encoding method according to the first embodiment.

First, when the recording medium 5 is inserted into the reader writer 41, the relating unit 451 reads the entire-volume information that is stored in the recording medium 5 via the reader writer 41 (Step S1A).

Next, on the basis of the number of multiple playback positions that are stored in the memory unit 42 and the entire-volume information that is acquired at Step S1A, the relating unit 451 relates multiple playback positions to the in-vivo images that are included in the in-vivo image group stored in the recording medium 5 and that are the same in number as the playback positions, as described below (Step S1B: a relating step).

FIG. 5 is a diagram that illustrates Step S1B.

Furthermore, in FIG. 5, a total frame number M (the entire-volume information) of in-vivo images F0 to F19 is 20, and a number N of multiple playback positions (pixels in the length direction of the time bar B) PT0 to PT4 is 5.

Specifically, the relating unit 451 uses the following Equation (1) to calculate an index value Kp that indicates the in-vivo image that is to be related to the p (p=0, 1, ..., N-1)th playback position and relates the in-vivo image with the frame number that is closest to the calculated index value Kp to the pth playback position.

$$Kp=(p \cdot (M-1))/(N-1) \qquad (1)$$

In the example illustrated in FIG. 5, an index value K0 (the index value that indicates the in-vivo image that is to be related to the 0th playback position PT0) is "0" according to Equation (1). Therefore, the relating unit 451 relates the in-vivo image F0 (indicated by a diagonal line in FIG. 5) with the frame number "0" to the 0th playback position PT0.

Furthermore, an index value K1 (the index value that indicates the in-vivo image that is to be related to the 1st playback position PT1) is "4.75" according to Equation (1). Therefore, the relating unit 451 relates the in-vivo image F5 (indicated by a diagonal line in FIG. 5) with the frame number "5" that is closest to the index value K1 (4.75) to the 1st playback position PT1.

In the same manner as described above, the other 2nd to 4th playback positions PT2 to PT4 are related to the in-vivo images F10, F14, F19 (indicated by diagonal lines in FIG. 5) with the frame numbers "10", "14", "19" that are closest to index values K2 to K4 that are calculated by using Equation (1).

Next, the encoding unit 452 sequentially reads the in-vivo image group, which is stored in the recording medium 5, via the reader writer 41 in the order of the frame number (Step S1C).

Then, the encoding unit 452 compresses and encodes (for example, the simple inter-frame predictive encoding illustrated in FIG. 11) multiple in-vivo images by using, as key frames, the in-vivo images (the in-vivo images F0, F5, F10, F14, F19 (indicated by diagonal lines) in the example of FIG. 5) that are related to multiple playback positions at Step S1B and by using the correlation between at least two images (Step S1D: an encoding step) and stores the compressed and encoded in-vivo image in the memory unit 42 (Step S1E).

The encoding unit 452 performs the above Steps S1C to S1E on up to the final image (the in-vivo image F19 in the example of FIG. 5) with the largest frame number in the in-vivo image group and, if it is performed on up to the final image (Step S1F: Yes), this process (the encoding method) is terminated.

[The image display method]

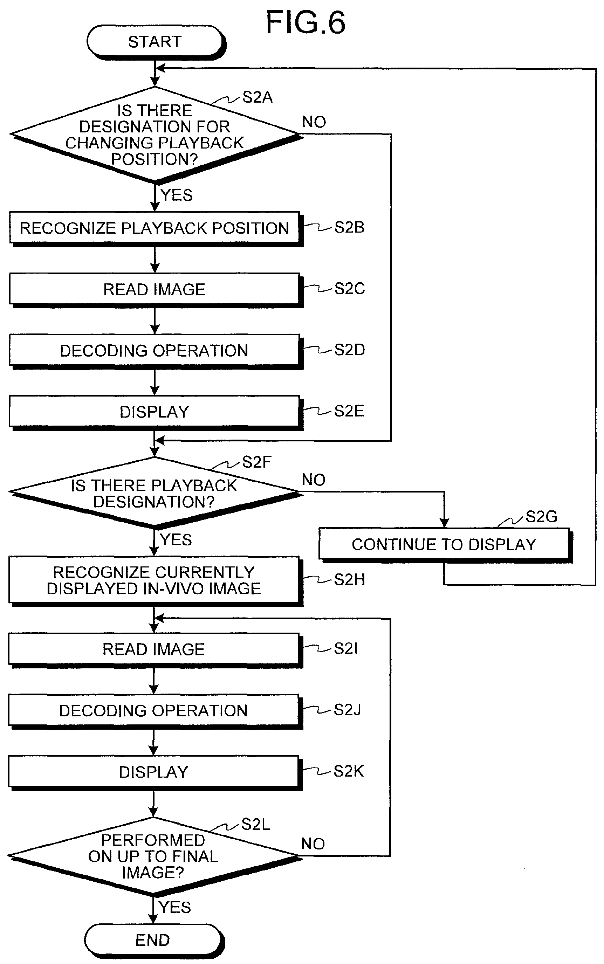

FIG. 6 is a flowchart that illustrates the image display method according to the first embodiment.

Furthermore, in the following, the playback screen W1 has been already displayed on the display unit 44. Moreover, any in-vivo image (on which a decoding operation has been performed by the decoding unit 453A) in the in-vivo image group, which is stored in the memory unit 42 at Step S1E, has been already displayed on the image display area FAr of the playback screen W1.

First, the control unit 45 determines whether there is a designation for changing the playback position in accordance with a user's operation on the input unit 43 (whether the slider SL is moved in accordance with a user's operation on the input unit 43 (Step S2A).

If it is determined that there is no designation for changing the playback position (Step S2A: No), the control unit 45 proceeds to Step S2F.

Conversely, if it is determined that there is a designation for changing the playback position (Step S2A: Yes), the decoding unit 453A recognizes the position (the playback position) of the slider SL on the time bar B (Step S2B).

Next, the decoding unit 453A reads the in-vivo image (key frame) that is included in the in-vivo image group, which is stored in the memory unit 42, and that is related to the playback position at Step S1B (Step S2C) and performs a decoding operation (for example, the decoding operation illustrated in FIG. 12) (Step S2D).

Then, the display controller 453B displays the in-vivo image, on which the decoding operation has been performed at Step S2D, in the image display area FAr of the playback screen W1 (Step S2E). Afterward, the control unit 45 proceeds to Step S2F.

At Step S2F, the control unit 45 determines whether there is a playback designation in accordance with a user's operation on the input unit 43 (whether the forward-direction playback icon A1 or the reverse-direction playback icon A2 is operated in accordance with a user's operation on the input unit 43).

If it is determined that there is no playback designation (Step S2F: No), the display controller 453B continues to display the in-vivo image that is currently displayed on the image display area FAr of the playback screen W1 (Step S2G).

Here, if the slider SL is not moved in accordance with a user's operation on the input unit 43 (Step S2A: No) and if the forward-direction playback icon A1 or the reverse-direction playback icon A2 is not operated (Step S2F: No), the above-described currently displayed in-vivo image is the in-vivo image (the in-vivo image that is a key frame or a non-key frame) that is already displayed on the image display area FAr of the playback screen W1 before Step S2A. Furthermore, if the slider SL is moved in accordance with a user's operation on the input unit 43 (Step S2A: Yes), the above-described currently displayed in-vivo image is the in-vivo image that is displayed on the image display area FAr of the playback screen W1 after Step S2B to S2E (the in-vivo image (the in-vivo image that is a key frame) that is related to the changed playback position).

Conversely, if it is determined that there is a playback designation (Step S2F: Yes), the decoding unit 453A recognizes the above-described currently displayed in-vivo image in the in-vivo image group that is stored in the memory unit 42 (Step S2H). Next, the decoding unit 453A sequentially reads the in-vivo image group, which is stored in the memory unit 42, from the in-vivo image that is recognized at Step S2H in the forward direction (if there is a forward-direction playback designation) in chronological order or in the reverse direction (if there is a reverse-direction playback designation) (Step S2I) and performs a decoding operation (for example, the decoding operation illustrated in FIG. 12) (Step S2J).

Then, the display controller 453B sequentially displays the in-vivo image on which the decoding operation has been performed at Step S2J on the image display area FAr of the playback screen W1 on a frame-to-frame basis (Step S2K).

Next, the control unit 45 determines whether the above Steps S2I to S2K have been performed on up to the final image (the in-vivo image F19 in the example of FIG. 5 if there is a forward-direction playback designation, and the in-vivo image F0 in the example of FIG. 5 if there is a reverse-direction playback designation) in the in-vivo image group (Step S2L).

Then, if it is determined that it has been performed on up to the final image (Step S2L: Yes), the control unit 45 terminates this process (the image display method).

According to the first embodiment that is described above, the image display device 4 includes the relating unit 451 and the encoding unit 452. Therefore, it is possible that all the key frames during compression encoding by the encoding unit 452 are the in-vivo images that are related to multiple playback positions. Specifically, even if various playback positions are designated, as the in-vivo image at the playback position is a key frame, the time it takes to display the in-vivo image can be relatively short.

Thus, with the image display device 4 according to the first embodiment, in-vivo images at various playback positions can be promptly displayed by using a moving-image compression technology that has a higher data compression rate compared to a still-image compression technology.

Furthermore, according to the first embodiment, on the basis of the number of multiple playback positions and the entire-volume information that indicates the volume of multiple images in entirety, the relating unit 451 relates multiple playback positions to the in-vivo images that are included in the in-vivo image group, which is stored in the recording medium 5, and that are the same in number as the playback positions.

Particularly, the entire-volume information is the total number of frames of the in-vivo image group, and the relating unit 451 calculates the index value Kp by using Equation (1) and relates the in-vivo image with the frame number that is closest to the index value Kp to the pth playback position.

Thus, in terms of the entire in-vivo image group, the in-vivo image at an appropriate position (frame number) can be related to the playback position. In other words, the in-vivo image at an appropriate position (frame number) can be a key frame.

Furthermore, according to the first embodiment, the time bar B, which is a time scale, is displayed on the playback screen W1, and the number of provided playback positions corresponds to the number of pixels in a length direction of the time bar B.

Here, in the capsule endoscope 2, the frame rate during capturing is fixed to a predetermined value.

Therefore, by relating the playback position to the in-vivo image using Equation (1), the in-vivo image at an appropriate position (time stamp) in the in-vivo image group can be related to the playback position in terms of the relationship with the time bar B (the time scale). In other words, the in-vivo image at an appropriate position (time stamp) can be a key frame.

(Second embodiment)

Next, an explanation is given of a second embodiment of the present invention.

In the following explanation, the same reference numerals are applied to the same configurations and steps as those in the above-described first embodiment, and their detailed explanations are omitted or simplified.

According to the above-described first embodiment, the frame rate during capturing by the capsule endoscope 2 is fixed to a predetermined value. Furthermore, at Step S1B, the relating unit 451 uses the total number of frames of the in-vivo image, which is the entire-volume information stored in the recording medium 5, to relate multiple playback positions to the in-vivo images that are included in the in-vivo image group, which is stored in the recording medium 5, and that are the same in number as the playback positions.

Conversely, according to the second embodiment, the frame rate during capturing by the capsule endoscope 2 is variable, and the total time (the time from when capturing is started by the capsule endoscope 2 until when it is terminated) of an in-vivo image group is used so that multiple playback positions are related to the in-vivo images that are included in the in-vivo image group, which is stored in the recording medium 5, and that are the same in number as the playback positions.

Furthermore, the configuration of the image display system according to the second embodiment is the same as the configuration in the above-described first embodiment.

An explanation is given below of only Step S1B according to the second embodiment.

The receiving device 3 according to the second embodiment causes the total time (the time stamp of the finally received in-vivo image) of the in-vivo image group to be stored as the entire-volume information in the recording medium 5.

Then, at Step S1B, the relating unit 451 according to the second embodiment relates multiple playback positions to the in-vivo images that are included in the in-vivo image group, which is stored in the recording medium 5, and that are the same in number as the playback positions, as described below.

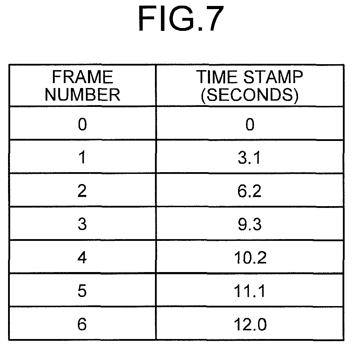

FIG. 7 is a diagram that illustrates Step S1B according to the second embodiment.

Specifically, FIG. 7 illustrates an example of the relevant information (the time stamp and the frame number) that is associated with the in-vivo image that is captured by the capsule endoscope 2.

Furthermore, FIG. 7 illustrates a case where the capsule endoscope 2 conducts capturing with the frame rate of 3.1 fps for the frame numbers "0" to "3" and conducts capturing with the frame rate of 0.9 fps for the frame number "4" and the subsequences. Moreover, in FIG. 7, the total number of frames of the in-vivo image is 7, and the total time of the in-vivo image group is 12.0 seconds (the time stamp of the in-vivo image with the largest frame number).

The relating unit 451 calculates an index value Kp' that indicates the in-vivo image that is to be related to the p (p=0, 1, ..., N-1)th playback position by using the following Equation (2), where the total time of the in-vivo image group is M' and the number of multiple playback positions is N, and relates the in-vivo image with the time stamp that is closest to the calculated index value Kp' to the pth playback position.

$$\mathrm{Kp'} = (\mathrm{p \cdot M'}) / (\mathrm{N-1}) \hspace{4cm} (2)$$

In the example illustrated in FIG. 7, if the number N of multiple playback positions is 3, an index value K0' (the index value that indicates the in-vivo image that is related to the 0th playback position) is "0" in accordance with Equation (2). Therefore, the relating unit 451 relates the in-vivo image with the frame number "0" that is the time stamp "0" to the 0th playback position.

Furthermore, an index value K1' (the index value that indicates the in-vivo image that is related to the 1st playback position) is "6" in accordance with Equation (2). Therefore, the relating unit 451 relates the in-vivo image with the frame number "2" that is the time stamp "6.2" to the 1st playback position.

Moreover, an index value K2' (the index value that indicates the in-vivo image that is related to the 2nd playback position) is "12.0" in accordance with Equation (2). Therefore, the relating unit 451 relates the in-vivo image with the frame number "6" that is the time stamp "12.0" to the 2nd playback position.

Then, the encoding unit 452 performs compression encoding with the in-vivo images that have the time stamps "0", "6.2", "12.0" as key frames at Step S1D.

The above-described second embodiment has the following advantage in addition to the same advantage as that of the above-described first embodiment.

With regard to relating of the playback position and the in-vivo image by using the total number of frames of the in-vivo image group as described above in the first embodiment, if the frame rate during capturing by the capsule endoscope 2 is variable, it is difficult to relate the playback position and the in-vivo image at an appropriate position (time stamp) in the in-vivo image group in terms of the relationship with the time bar B (time scale).

Conversely, according to the second embodiment, the relating unit 451 relates the playback position to the in-vivo image by using the total time of the in-vivo image group.

Therefore, even if the frame rate during capturing is variable, it is possible to relate the playback position and the in-vivo image at an appropriate position (time stamp) in the in-vivo image group in terms of the relationship with the time bar B (time scale). In other words, the in-vivo image at an appropriate position (time stamp) can be a key frame.

(Third embodiment)

Next, a third embodiment of the present invention is explained.

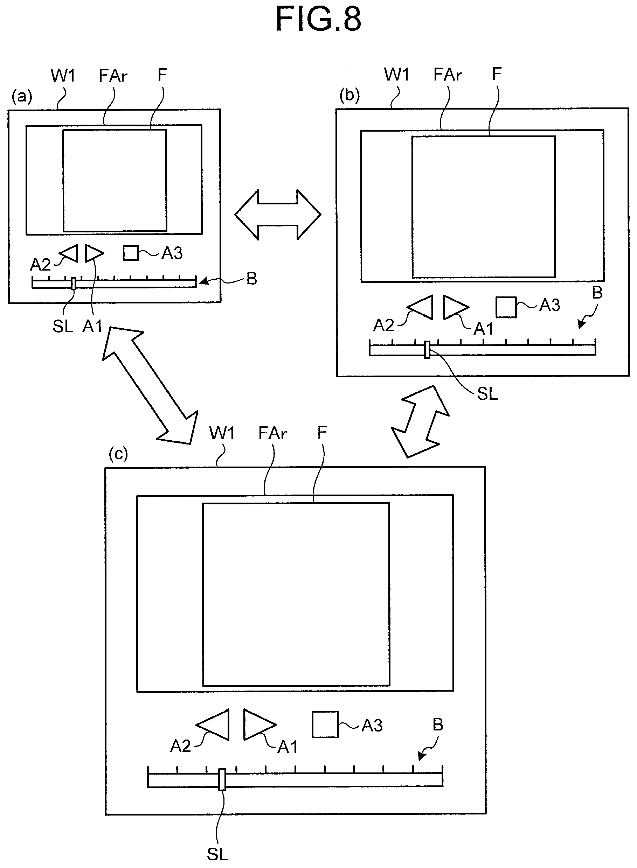

FIG. 8 is a diagram that illustrates an example of the playback screen W1 according to the third embodiment of the present invention.

In the following explanation, the same reference numerals are applied to the same configurations and steps as those in the above-described first embodiment, and their detailed explanations are omitted or simplified.

According to the third embodiment, as illustrated in FIG. 8, the display controller 453B can change the resolution of the playback screen W1 that is displayed on the display unit 44 in accordance with a user's operation on the input unit 43 (in the example of FIG. 8, it can be changed to a resolution R1 (FIG. 8(a)), a resolution R2 (FIG. 8(b)), or a resolution R3 (FIG. 8(c))).

Furthermore, the configuration of the image display system according to the third embodiment is the same configuration as that in the above-described first embodiment.

An explanation is given below of only Step S1B according to the third embodiment in a case where the resolution of the playback screen W1 is changed as in the example illustrated in FIG. 8.

As described above, the number of playback positions corresponds to the number of pixels in the length direction of the time bar B.

Furthermore, if the resolution of the playback screen W1 is changed as illustrated in FIG. 8, the number of pixels in the length direction of the time bar B is changed and therefore the number of playback positions is also changed.

Therefore, according to the third embodiment, the number of pixels (the number of playback positions) in the length direction of the time bar B is previously stored in the memory unit 42 with respect to each of the resolutions R1 to R3 of the playback screen W1.

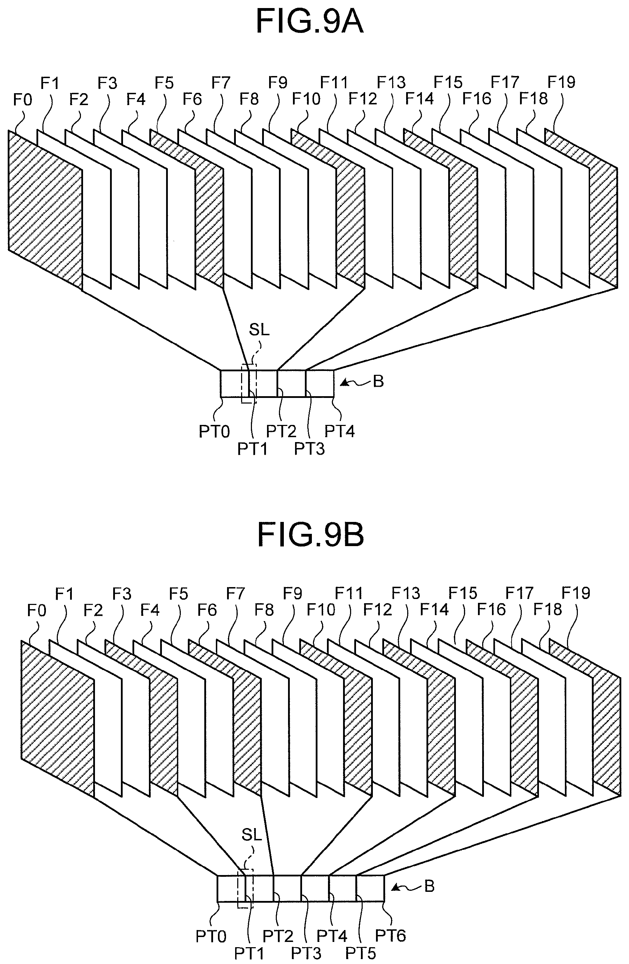

FIG. 9A to FIG. 9C are diagrams that illustrate Step S1B according to the third embodiment.

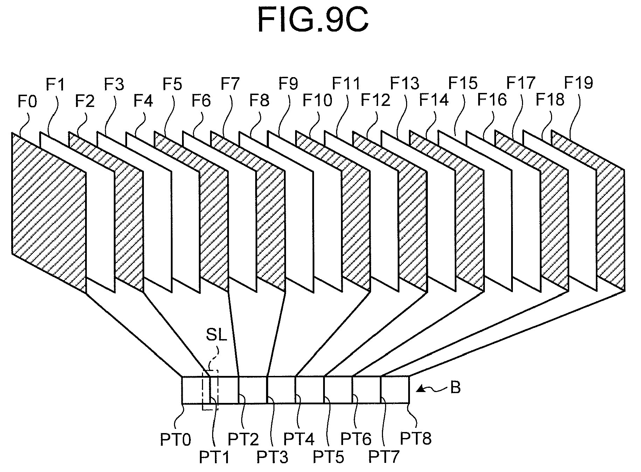

Furthermore, as is the case with FIG. 5, the total frame number M (the entire-volume information) of the in-vivo image is 20 in FIG. 9A to FIG. 9C. Moreover, FIG. 9A is a diagram that corresponds to a case where the resolution of the playback screen W1 is the resolution R1 as illustrated in FIG. 8 (a), and the number of the playback positions PT0 to PT4 is five (as is the case with FIG. 5). FIG. 9B is a diagram that corresponds to a case where the resolution of the playback screen W1 is the resolution R2 as illustrated in FIG. 8(b), and the number of the playback positions PT0 to PT6 is 7. FIG. 9C is a diagram that corresponds to a case where the resolution of the playback screen W1 is the resolution R3 as illustrated in FIG. 8(c), and the number of the playback positions PT0 to PT8 is 9.

Specifically, at Step S1B, the relating unit 451 reads, from the memory unit 42, the number of playback positions that correspond to the resolution of the playback screen W1 that is displayed on the display unit 44 and, as is the case with the above-described first embodiment, relates multiple playback positions to the in-vivo images that are included in the in-vivo image group, which is stored in the recording medium 5, and that are the same in number as the playback positions on the basis of the number of playback positions and the entire-volume information that is acquired at Step S1A.

In the example of FIG. 8(a) and FIG. 9A, the relating unit 451 reads, from the memory unit 42, the number "5" of playback positions that correspond to the resolution R1 of the playback screen W1 that is displayed on the display unit 44. Furthermore, the relating unit 451 calculates the index value Kp by using Equation (1) on the basis of the total frame number M (M=20) of the in-vivo image and the number N (N=5) of the playback positions PT0 to PT4. Then, the relating unit 451 relates the 0th to 4th playback positions PT0 to PT4 to the in-vivo images F0, F5, F10, F14, F19 (indicated by diagonal lines in FIG. 9A) with the frame numbers "0", "5", "10", "14", "19".

In the example of FIG. 8(b) and FIG. 9B, the relating unit 451 reads, from the memory unit 42, the number "7" of playback positions that correspond to the resolution R2 of the playback screen W1 that is displayed on the display unit 44. Furthermore, the relating unit 451 calculates the index value Kp by using Equation (1) on the basis of the total frame number M (M=20) of the in-vivo image and the number N (N=7) of the playback positions PT0 to PT6. Then, the relating unit 451 relates the 0th to 6th playback positions PT0 to PT6 to the in-vivo images F0, F3, F6, F10, F13, F16, F19 (indicated by diagonal lines in FIG. 9B) with the frame numbers "0", "3", "6", "10", "13", "16", "19".

In the example of FIG. 8(c) and FIG. 9C, the relating unit 451 reads, from the memory unit 42, the number "9" of playback positions that correspond to the resolution R3 of the playback screen W1 that is displayed on the display unit 44. Furthermore, the relating unit 451 calculates the index value Kp by using Equation (1) on the basis of the total frame number M (M=20) of the in-vivo image and the number N (N=9) of the playback positions PT0 to PT8. Then, the relating unit 451 relates the 0th to 8th playback positions PT0 to PT8 to the in-vivo images F0, F2, F5, F7, F10, F12, F14, F17, F19 (indicated by diagonal lines in FIG. 9C) with the frame numbers "0" "2" "5" "7" "10", "12", "14", "17", "19".

The above-described third embodiment has the following advantage in addition to the same advantage as the above-described first embodiment.

According to the third embodiment, the number of playback positions that correspond to the resolution of the playback screen W1 is stored in the memory unit 42, and the relating unit 451 reads, from the memory unit 42, the number of playback positions that correspond to the resolution of the playback screen W1 that is displayed on the display unit 44 and uses the number of playback positions to relate the playback positions to the in-vivo images.

Therefore, even if the resolution of the playback screen W1 is changed, the in-vivo image at an appropriate position (the frame number, the time stamp) in the in-vivo image group can be related to the playback position. In other words, the in-vivo image at an appropriate position (the frame number, the time stamp) can be a key frame.

(Other embodiments)

Heretofore, the embodiments for implementing the present invention have been explained; however, the present invention should not be limited to only the above-described first to third embodiments.

According to the above-described first to third embodiments, each pixel in the length direction of the time bar B that is displayed on the display unit 44 is a playback position; however, this is not a limitation.

For example, a configuration may be such that what is called a random playback is enabled by sliding or rotating a mechanical switch, such as a slide switch that is slidable in multiple steps or a rotary encoder that is rotatable in multiple steps, and a slide position or a rotation position of the mechanical switch may be a playback position.

According to the above-described first to third embodiments, the number of in-vivo images that are the key frames for compression encoding is the same as the number of playback positions; however, this is not a limitation, and the number of in-vivo images that are the key frames may be larger than the number of playback positions. That is, in addition to the in-vivo images that are related to the playback positions, some of the in-vivo images that are not related to the playback positions may be key frames.

According to the above-described first to third embodiments, the simple inter-frame predictive encoding method is used as a moving-image compression technology; however, this is not a limitation, and other methods, such as a motion-compensated inter-frame predictive encoding method, may be used if the method uses key frames.

According to the above-described first to third embodiments, a configuration is such that the receiving device 3 stores the entire-volume information in the recording medium 5; however, this is not a limitation, and it is possible to use a configuration such that the relating unit 451 refers to image data (in-vivo image) in the recording medium 5 via the reader writer 41 and acquires the entire-volume information that is the total number of frames of the in-vivo image group or the total time of the in-vivo image group.

According to the above-described first to third embodiments, a configuration is such that the image display device according to the present invention displays an in-vivo image that is captured by the capsule endoscope 2; however, this is not a limitation, and a configuration may be such that other images are displayed.

Reference Signs List

| | |
|---|---|
| 1 | IMAGE DISPLAY SYSTEM |
| 2 | CAPSULE ENDOSCOPE |
| 3 | RECEIVING DEVICE |
| 3a TO 3h | RECEIVING ANTENNA |
| 4 | IMAGE DISPLAY DEVICE |
| 5 | RECORDING MEDIUM |
| 41 | READER/WRITER |
| 42 | MEMORY UNIT |
| 43 | INPUT UNIT |
| 44 | DISPLAY UNIT |
| 45 | CONTROL UNIT |

| 100 | SUBJECT |
| 451 | RELATING UNIT |
| 452 | ENCODING UNIT |
| 453 | PLAYBACK PROCESSING UNIT |
| 453A | DECODING UNIT |
| 453B | DISPLAY CONTROLLER |
| A1 | FORWARD-DIRECTION PLAYBACK ICON |
| A2 | REVERSE-DIRECTION PLAYBACK ICON |
| A3 | TEMPORARY STOP ICON |
| B | TIME BAR |
| F0 TO F19 | IN-VIVO IMAGE |
| FAr | IMAGE DISPLAY AREA |
| Fd1 TO Fd3 | SUBTRACTED IMAGE |
| $F_K$ | KEY FRAME |
| $F_S$ | NON-KEY FRAME |
| PT0 TO PT8 | PLAYBACK POSITION |
| W1 | PLAYBACK SCREEN |

**Claims**

1. An image display device that performs a playback operation to sequentially display multiple images on a display unit, the image display device comprising:

   a designation receiving unit that receives a designation of any playback position among multiple playback positions for starting the playback operation;
   a relating unit that relates the multiple playback positions to the number of the images that are included in the multiple images and that correspond to the number of the multiple playback positions; and
   an encoding unit that compresses and encodes the multiple images by using, as key frames, images that are included in the multiple images and that are related to the multiple playback positions by the relating unit and by using a correlation between at least two of the images.

2. The image display device according to claim 1, wherein the relating unit uses the number of the multiple playback positions and entire-volume information that indicates a volume of the multiple images in entirety to relate the multiple playback positions to the number of the images that are included in the multiple images and that corresponds to the number of the multiple playback positions.

3. The image display device according to claim 2, wherein
   the entire-volume information is the number of frames of the multiple images in entirety, and
   the relating unit calculates an index value Kp by using $Kp=(p \cdot (M-1))/(N-1)$, where the number of frames is M, the number of the multiple playback positions is N, and an index value that indicates an image that is related to the p (p=0, 1, ..., N-1)th playback position is Kp, and relates an image with a frame number that is closest to the index value Kp to the pth playback position.

4. The image display device according to claim 2, wherein
   the multiple images are images that are acquired in chronological order and that are associated with an elapsed time after corresponding acquisition is started,
   the entire-volume information is a total time after the acquisition of the multiple images is started until terminated, and
   the relating unit calculates an index value Kp' by using $Kp'=(p \cdot M')/(N-1)$, where the total time is M', the number of the multiple playback positions is N, and the index value that indicates an image that is related to the p (p=0, 1, ..., N-1)th playback position is Kp', and relates an image that is associated with the elapsed time that is closest to the index value Kp' to the pth playback position.

5. The image display device according to any one of claims 1 to 4, comprising:

   a decoding unit that decodes the multiple images that are compressed and encoded by the encoding unit; and
   a display controller that performs the playback operation on the multiple images that are decoded by the decoding unit and that displays, on the display unit, a bar that indicates a volume of the multiple images in entirety, wherein

the number of the playback positions provided corresponds to the number of pixels in a length direction of the bar that is displayed on the display unit.

6. The image display device according to claim 5, wherein
the display controller displays, on the display unit, a playback screen that includes the image and the bar and is capable of changing a resolution of the playback screen to multiple resolutions,
the image display device includes a memory unit that stores the number of the playback positions that correspond to each of the multiple resolutions, and
the relating unit reads, from the memory unit, the number of the playback positions that correspond to the resolution of the playback screen that is displayed on the display unit and uses the number of the playback positions to relate the multiple playback positions to the number of the images that are included in the multiple images and that correspond to the number of the multiple playback positions.

7. An encoding method performed by an image display device that performs a playback operation to sequentially display multiple images on a display unit, the encoding method comprising:

a relating step of relating multiple playback positions for starting the playback operation to the number of the images that are included in the multiple images and that correspond to the number of the multiple playback positions; and
an encoding step of compressing and encoding the multiple images by using, as key frames, images that are included in the multiple images and that are related to the multiple playback positions at the relating step and by using a correlation between at least two of the images.

8. An encoding program that causes the image display device to execute the encoding method according to claim 7.

# FIG.1

CAPSULE ENDOSCOPE
2

100
SUBJECT

RECEIVING
ANTENNA
3a

3b    3c

RECEIVING
DEVICE
3

3d    3e

3f    3g    3h

1

IMAGE DISPLAY
DEVICE
4

5
RECORDING
MEDIUM

EP 3 046 327 A1

# FIG.2

| | 44 |
|---|---|
| | DISPLAY UNIT |

41
READER
WRITER

RECORDING
MEDIUM — 5

45
CONTROL UNIT

RELATING UNIT — 451

ENCODING UNIT — 452

PLAYBACK
PROCESSING UNIT — 453

DECODING UNIT — 453A

DISPLAY
CONTROLLER — 453B

43
INPUT
UNIT

42
MEMORY UNIT

4

# FIG.3

# FIG.4

```
           START
             │
             ▼
  READ ENTIRE-VOLUME INFORMATION        ⟿ S1A
             │
             ▼
  RELATE PLAYBACK POSITIONS TO IMAGES    ⟿ S1B
             │
             ▼  ◄───────────────┐
        READ IMAGE              ⟿ S1C    │
             │                           │
             ▼                           │
         ENCODE                 ⟿ S1D    │
             │                           │
             ▼                           │
         STORE                  ⟿ S1E    │
             │                           │
             ▼        ⟿ S1F              │
    PERFORMED ON UP            NO         │
     TO FINAL IMAGE? ──────────────────┘
             │
           YES
             ▼
           END
```

# FIG.5

F0 F1 F2 F3 F4 F5 F6 F7 F8 F9 F10 F11 F12 F13 F14 F15 F16 F17 F18 F19

SL

B

PT0  PT1  PT2  PT3  PT4

# FIG.6

```
                    START

                     │
          ┌──────────▼──────────┐
          │     IS THERE        │    S2A
          │  DESIGNATION FOR    │─── NO ───┐
          │ CHANGING PLAYBACK   │          │
          │    POSITION?        │          │
          └──────────┬──────────┘          │
                   YES                      │
                     │                      │
      ┌──────────────▼──────────────┐       │
      │ RECOGNIZE PLAYBACK POSITION │  S2B  │
      └──────────────┬──────────────┘       │
                     │                      │
      ┌──────────────▼──────────────┐       │
      │        READ IMAGE           │  S2C  │
      └──────────────┬──────────────┘       │
                     │                      │
      ┌──────────────▼──────────────┐       │
      │     DECODING OPERATION      │  S2D  │
      └──────────────┬──────────────┘       │
                     │                      │
      ┌──────────────▼──────────────┐       │
      │        DISPLAY              │  S2E  │
      └──────────────┬──────────────┘       │
                     │◄─────────────────────┘
          ┌──────────▼──────────┐   S2F
          │   IS THERE PLAYBACK │─── NO ───┐
          │    DESIGNATION?     │          │
          └──────────┬──────────┘   ┌──────▼──────────┐  S2G
                   YES              │ CONTINUE TO     │
                     │              │   DISPLAY       │
      ┌──────────────▼──────────────┐└──────┬─────────┘
      │ RECOGNIZE CURRENTLY         │  S2H   │
      │ DISPLAYED IN-VIVO IMAGE     │        │
      └──────────────┬──────────────┘        │
                     │◄────────────┐         │
      ┌──────────────▼──────────────┐  S2I   │
      │        READ IMAGE           │        │
      └──────────────┬──────────────┘        │
                     │                       │
      ┌──────────────▼──────────────┐  S2J   │
      │     DECODING OPERATION      │        │
      └──────────────┬──────────────┘        │
                     │                       │
      ┌──────────────▼──────────────┐  S2K   │
      │        DISPLAY              │        │
      └──────────────┬──────────────┘        │
          ┌──────────▼──────────┐   S2L       │
          │    PERFORMED        │─── NO ──────┘
          │  ON UP TO FINAL     │
          │     IMAGE?          │
          └──────────┬──────────┘
                   YES
                     │
                    END
```

# FIG.7

| FRAME NUMBER | TIME STAMP (SECONDS) |
|:---:|:---:|
| 0 | 0 |
| 1 | 3.1 |
| 2 | 6.2 |
| 3 | 9.3 |
| 4 | 10.2 |
| 5 | 11.1 |
| 6 | 12.0 |

# FIG.8

# FIG.9A

# FIG.9B

# FIG.9C

# FIG.10

| F0 | F1 | F2 | F3 |
|---|---|---|---|
| STORAGE UNIT | STORAGE UNIT | STORAGE UNIT | STORAGE UNIT |

# FIG.11

F0(F$_K$)   F1(F$_S$)   F2(F$_S$)   F3(F$_S$)

Fd1   Fd2   Fd3

STORAGE UNIT   STORAGE UNIT   STORAGE UNIT   STORAGE UNIT

# FIG.12

F0(F$_K$)　　　　　F1(F$_S$)　　　　　F2(F$_S$)　　　　　F3(F$_S$)

F0(F$_K$)　　　　　Fd1　　　　　Fd2　　　　　Fd3

STORAGE UNIT　　　STORAGE UNIT　　　STORAGE UNIT　　　STORAGE UNIT

## INTERNATIONAL SEARCH REPORT

International application No.

PCT/JP2014/065000

A. CLASSIFICATION OF SUBJECT MATTER
*H04N19/107(2014.01)i, H04N19/162(2014.01)i, H04N19/172(2014.01)i*

According to International Patent Classification (IPC) or to both national classification and IPC

B. FIELDS SEARCHED

Minimum documentation searched (classification system followed by classification symbols)
H04N19/00-19/98, H04N5/76-5/956

Documentation searched other than minimum documentation to the extent that such documents are included in the fields searched
Jitsuyo Shinan Koho        1922-1996   Jitsuyo Shinan Toroku Koho   1996-2014
Kokai Jitsuyo Shinan Koho  1971-2014   Toroku Jitsuyo Shinan Koho   1994-2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 10-294953 A  (Aisin AW Co., Ltd.), 04 November 1998 (04.11.1998), entire text; all drawings (Family: none) | 1-8 |
| A | JP 2008-136759 A  (Pentax Corp.), 19 June 2008 (19.06.2008), entire text; all drawings (Family: none) | 1-8 |
| A | JP 2002-112169 A  (Minolta Co., Ltd.), 12 April 2002 (12.04.2002), entire text; all drawings & US 2002/0036632 A1 | 1-8 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 14 August, 2014 (14.08.14) | 02 September, 2014 (02.09.14) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2014/065000

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2005-20203 A  (Canon Inc.),<br>20 January 2005 (20.01.2005),<br>entire text; all drawings<br>(Family: none) | 1-8 |
| A | JP 2009-38680 A  (Toshiba Corp.),<br>19 February 2009 (19.02.2009),<br>entire text; all drawings<br>& US 2009/0034806 A1    & CN 101360198 A | 1-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5197892 B